# EUROPEAN PATENT SPECIFICATION

(11) **EP 3 849 415 B1**
(45) Date of publication and mention of the grant of the patent: **16.04.2025**
(21) Application number: 19860380.5
(22) Date of filing: 16.09.2019
(51) Int. Cl.: A61B 5/097, A61B 5/08, A61B 5/087, A61B 5/00

(54) **SELF-USE OSCILLOMETRY DEVICE**
OSZILLOMETER ZUM EIGENGEBRAUCH
DISPOSITIF D'OSCILLOMÉTRIE À USAGE PERSONNEL

(30) Priority: 14.09.2018 US 201862731424 P
(43) Date of publication of application: 21.07.2021
(73) Proprietor: Thorasys Thoracic Medical Systems Inc., Montréal, Québec H2V 4L5 (CA)
(72) Inventor: SCHUESSLER, Thomas Florian, Montreal, Québec H2V 4L5 (CA); CHOW SUN FAT, Kim Fur, Montreal, Québec H2V 4L5 (CA); POSADA ESTEFAN, Lucas, Montreal, Québec H2V 4L5 (CA); DRAPEAU, Guy, Montreal, Québec H2V 4L5 (CA); JUTRAS, Sebastien, Montreal, Québec H2V 4L5 (CA); CHICATUN, Florencia, Montreal, Québec H2V 4L5 (CA)
(74) Representative: Rummler, Felix
(86) International application number: PCT/CA2019/051308
(87) International publication number: WO 2020/051718

(56) References cited:
- EP-B1- 2 598 026
- WO-A1-2009/133561
- CN-A- 108 095 724
- US-A1- 2013 150 747
- US-A1- 2017 135 603
- US-A1- 2017 333 652
- NA: "tremoflo C-100 Airwave Oscillometry System - User Manual", 13 July 2018 (2018-07-13), Montreal, Quebec H2V 4L5, pages 1 - 92, XP055784287, Retrieved from the Internet <URL:https://www.lok.training/wp-content/uploads/2020/11/101648-Rev-AI-tremoflo-C100-User-Manual.pdf> [retrieved on 20210310]

## Description

### CROSS-REFERENCE TO RELATED APPLICATION

The present applications claims the priority of United States Patent Application No. 62/731,424, filed on September 14, 2018.

### TECHNICAL FIELD

The present disclosure pertains to oscillometry measurements (a.k.a., forced oscillation technique (FOT)) and equipment as used in the assessment and monitoring of respiratory mechanics and pulmonary function for example in the context of asthma or chronic obstructive pulmonary disease (COPD).

### BACKGROUND OF THE ART

Unlike conventional techniques to measure lung functions such as spirometry or peak flow, oscillometry does not rely on a voluntary effort by the user (meaning, in this context, the subject or patient to be measured, in contrast to a person overseeing the measurement) to perform a specific manoeuvre. Rather, oscillometry measurements are obtained during regular quiet breathing, making the technique natural for the user.

During an oscillometry measurement, a gentle oscillatory waveform generated by an oscillometry device is superimposed on the quiet breathing of the user. The frequencies of interest contained in the oscillatory waveform are situated above the power spectrum of the spontaneous breathing, so that the oscillatory and breathing components contained in the recordings can be separated using numerical techniques such as Fourier transforms and time-frequency analysis.

One constraint associated with oscillometry measurements is that the users, typically breathing through a mouthpiece, must support their cheeks and mouth floor with their hands to ensure that the oscillatory energy generated by the oscillometry device reaches the lungs and does not get lost in the softer, compliant tissues of the upper airways. Expressed in more technical terms, the hands must be used to increase the shunt impedance presented by the upper airway walls that would otherwise be too low and short-circuit the oscillation.

Because both of the user's hands are used to support the left and right cheeks, the user may not be able to simultaneously hold a handheld, portable oscillometry device. Therefore, oscillometry devices must be either mounted to a physical support such as a desktop or trolley, or if portable, an independent operator is required to hold the device during the measurement while the users support their cheeks.

Consequently, the existing portable devices may not lend themselves to self-assessment "anywhere", i.e., in a manner adapted to the user's lifestyle, as is the case for example for blood pressure monitoring. Another person is often needed to hold and operate the device, therefore such measurements can no longer be considered self-assessment.

European Patent No. 2598026 describes plates of slightly concave substantially triangular shape which are positioned on the user's cheeks to exert a slight pressure, intrinsic of the plates, and hold them still. The plates are intended to replace manual cheek support, which means that they must exert a pressure onto the user's cheeks that is similar to the pressure that would be exerted by the user's hands during manual cheek support. These plates possess significant rigidity in a direction normal to the cheek skin surface. The weight of the plates is supported by their attachment to the device, which must in turn be supported by an appropriate structure or independent operator.

US 2017/135603 A1 describes an apparatus comprising an airflow measurement tube with an open end configured to form an airtight seal with an airway of a subject, a dental device coupled with the airflow measurement tube and configured to position a mandible of the subject with respect to a maxilla of the subject, and a sensor coupled with the airflow measurement tube and configured to measure at least one of an airflow through the airflow measurement tube or a pressure within the airflow measurement tube.

US 2017/333652 A1 describes an apparatus to generate pressure support ventilation, comprising a sensor adapted to measure a respiratory parameter, a flow generator adapted for coupling with a patient respiratory interface, the flow generator configured to provide a flow of breathable gas for pressure support ventilation to the patient respiratory interface, and a controller, coupled to the one sensor and the flow generator, the controller configured to control the pressure support ventilation with the flow generator.

### SUMMARY

An aim of the present disclosure is to provide a self-use oscillometry device that addresses issues related to the prior art.

A further aim of the present disclosure is to provide a portable oscillometry device that may be physically supported by a user without compromising, or even while ensuring proper cheek support and device positioning.

The present invention is defined in claim 1. Selected optional features are recited in the sub-claims.

In accordance with a first embodiment of the present disclosure, there is provided an oscillometry device comprising: a casing, a user portion forming a conduit entering into the casing, the conduit adapted to receive a breath of a user, an oscillometry measurement system operatively connected to the conduit in the casing and adapted to produce oscillometry measurement signals from the breath of the user, and a user support interface projecting from the casing in a common direction with the user portion, the user support interface vertically supporting the oscillometry device relative to a user when the user has his or her mouth on the user portion, the user support interface being made of a flexible deformable material.

The user support interface includes a pair of handles projecting from the casing.

Still further in accordance with the first embodiment, the handles are elongated strips of the flexible deformable material.

Still further in accordance with the first embodiment, the elongated strips form loops.

Still further in accordance with the first embodiment, the loops are sized to receive four fingers or a palm of a user.

Still further in accordance with the first embodiment, for instance, at least one finger loop is on at least one of the lateral surfaces of the elongated strips.

Still further in accordance with the first embodiment, for instance, at least one pressure sensor is on at least one of the handles producing a signal indicative of a pressure applied thereon.

Still further in accordance with the first embodiment, for instance, at least one inertial sensor on the casing produces a signal indicative of an orientation of the oscillometry device.

In an embodiment not covered by the claims, for instance, the user support interface includes at least one head strap.

Still further in accordance with the first embodiment, for instance, the handles have a height of at most 4.0 cm in a face contacting end.

Still further in accordance with the first embodiment, for instance, a height to thickness ratio for the face contacting end of the handles is of at least 10.

In accordance with an unclaimed example of the present disclosure, there is provided an oscillometry device comprising: a casing, a user portion projecting in a user direction and forming a conduit entering into the casing, the conduit adapted to receive a breath of a user, the user portion having a central axis, the central axis being normal to a reference plane of the user portion, the conduit plane being at an end of the user portion in the user direction, an oscillometry measurement system operatively connected to the conduit in the casing and adapted to produce oscillometry measurement signals from the breath of the user, and a user support interface projecting from the casing and having handles extending beyond the reference plane in the user direction, the user support interface vertically supporting the oscillometry device relative to a user when the user has his or her mouth on the user portion, the user support interface being made of a flexible deformable material, the handles being positioned laterally of the central axis and vertically located at least partially in a range of +5 cm to -5.0 cm of the central axis.

Further in accordance with the example, for instance, the handles are elongated strips of the flexible deformable material.

Still further in accordance with the example, for instance, the elongated strips form loops.

Still further in accordance with the example, for instance, the loops are sized to receive four fingers or a palm of a user.

Still further in accordance with the example, for instance, at least one finger loop is on the lateral surfaces of the elongated strips.

Still further in accordance with the example, for instance, at least one pressure sensor on the handles produces a signal indicative of a pressure applied thereon.

Still further in accordance with the example, for instance, at least one inertial sensor on the casing produces a signal indicative of an orientation of the oscillometry device.

Still further in accordance with the example, for instance, the handles are part of a head strap.

Still further in accordance with the example, for instance, the handles have a height of at most 4.0 cm in a face contacting end.

Still further in accordance with the example, for instance, a height to thickness ratio for the face contacting end of the handles is of at least 10.

Still further in accordance with the example, for instance, ends of the handle extend beyond the reference plane by at least 3.0 cm.

Still further in accordance with the example, for instance, ends of the handle extend are located at a distance from 3.0 cm to 12.0 cm beyond the reference plane.

### DESCRIPTION OF THE DRAWINGS

Fig. 1 is a schematic view of a self-use oscillometry device in accordance with the present disclosure;
Fig. 2 is a schematic view with block diagram showing an interior of the oscillometry device of Fig. 1 and an embodiment of user support interface for the oscillometry device;
Fig. 3 is a schematic view showing another embodiment of user support interface for the oscillometry interface;
Fig. 4 is a schematic view showing another embodiment of user support interface for the oscillometry interface;
Fig. 5 is a schematic view showing another embodiment of user support interface for the oscillometry interface;
Fig. 6 is a schematic view showing another embodiment of user support interface for the oscillometry interface;
Fig. 7 is a schematic view showing another embodiment of user support interface for the oscillometry interface; and
Fig. 8 is a schematic view showing another embodiment of user support interface for the oscillometry interface.

### DETAILED DESCRIPTION

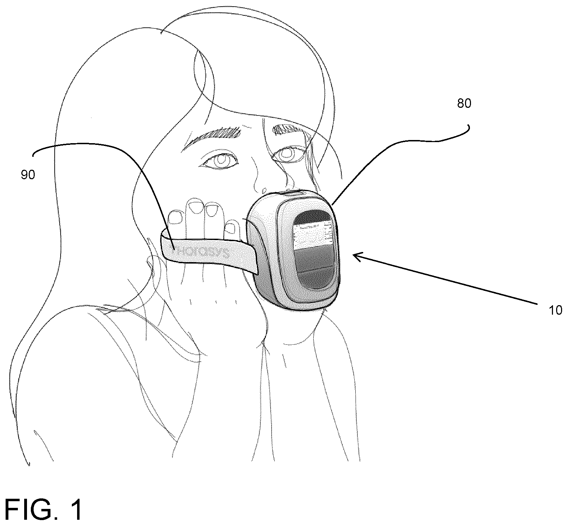

Referring to the drawings and more particularly to Fig. 1, there is shown a self-use oscillometry device 10 in accordance with the present disclosure. The oscillometry device 10 may be described as being "self-use", "self-assessment", "portable", among other expressions, to express the fact that the device 10 is a small, compact and portable oscillometry device suitable for user self-assessment and self-monitoring without requiring the presence and assistance of another human being, in certain circumstances (e.g., age of the user, physical autonomy, etc). For further clarity, the notion of portability in this context means that the device 10 may easily and comfortably be used in a confined space, e.g., on a plane or train, and transported in a small personal luggage item such as a small backpack or airplane carry-on luggage. Fig. 1 shows a schematic view of self-use of the oscillometry device 10.

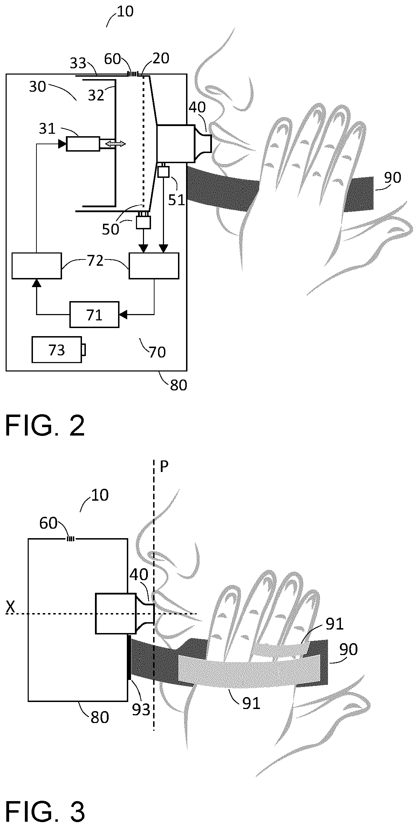

Referring to Fig. 2, an embodiment of the device 10 is shown having a casing 80 including the various hardware, electronic and software components to operate self-use oscillometry measurements. The casing 80 is of limited volume to allow the self-use described above. For example, in practical terms, this means that the oscillometry device 10 may fit into an limited volume envelope (e.g., not exceeding 2 litres), be lightweight (e.g., no more than 1 kg), and/or be independently powered and operable. The oscillometry device 10 may have a breathing conduit 20 projecting out of the casing 80, and defining an air passage into the casing 80. The breathing conduit 20 is connected a compact oscillatory flow source 30, which is illustrated as a linear actuator 31 connected to a piston 32 that is moving in a housing 33. As an example among others, the compact oscillatory flow source 30 may equally be a membrane driven by a linear oscillator, a loudspeaker, a rotary fan or a valve connected to a small cylinder containing pressurized air, just to name a few.

The conduit 20 has a user port 40 at a free end. The user port 40 may or may not be releasably fitted with a suitable interface to the user's airway opening, such as a mouthpiece. A flow meter 50 is located between the oscillatory flow source 30 and the user port 40, and is in fluid communication with the conduit 20, to measure the airflow into and out of the user's airway opening. The fluid communication may be qualitied as operative connection, in that the airflow into the user port 40 and conduit 20 reaches the oscillometry measurement system, in operative connection. The flow meter 50 may include screen, honeycomb, ultrasonic, variable orifice or venturi pneumotachographs, just to name a few. Between the flow meter 50 and the user port 40 is a pressure meter 51 to measure the pressure proximal to the user's airway opening.

Respiratory system input impedance and other indices of respiratory mechanics and lung function may be calculated from the data recorded by the flow meter 50 and the pressure meter 51, and a dynamic characterization of the air passages between the user and the site of measurement, i.e. the user port 40 and the portion of the conduit 20 situated between the user port 40 and the flow meter 50, may be used to enhance the accuracy of the impedances and indices calculated.

The conduit 20 may further be in fluid communication with an atmosphere port 60 with a deterministic impedance to air flow that is sufficiently low to prevent undue loading of the user's breathing, but sufficiently high to prevent short-circuiting of the oscillatory waveform.

The device 10 may further include all required electronics 70, including at least one processing module 71 with one or more processors with memory for data storage, communication facilities etc., peripherals 72 such as A/D and D/A converters, signal amplifiers, filters and oscillator driver, and an energy source 73 such as a battery, i.e. all components that are needed to generate the oscillation, record the measurements, store the results and transmit them to another device such as a computer, tablet, smart phone, telemedicine server or central database, just to name a few. The processing module 71 may be in the form of a non-transitory computer-readable memory communicatively coupled to the processing unit and comprising computer-readable program instructions executable by the processing unit for performing and asses oscillometry measurements. In another embodiment, the device may communicate wirelessly with a computer or portable electronic device, and some of the processing or user interface functionality described above may be located on this portable electronic device.

All of the above components are physically firmly attached to each other in the compact and sufficiently lightweight enclosed casing 80, which is assembled for use but which may be configured to be disassembled for cleaning and maintenance. This may allow access to the oscillometry measuring system, i.e., the various components described above, including hardware, electronics and software, operation oscillometry measurements. Further attached to the casing 80 is a user support interface 90. According to an embodiment shown in Fig. 2, the user support interface 90 may take the form of one or two pliable, anatomically and ergonomically shaped grips 90 (one shown in Fig. 2, but the right-hand side being a mirror image of the left-hand side of Fig. 2) that allow the user to pick up the oscillometry device 10 by these grips 90, bring it close to their mouth, and ultimately position the oscillometry device 10 such that the user port 40 is connected to the airway opening (e.g. the mouthpiece is in the mouth). The grips 90 may also be known as handles. In some embodiments, part of the weight of the device 10 may also be supported by the user port 40 (or, more specifically, the user's bite on the mouthpiece) once the device is in place. The grips 90 may be defined as elongated flat strips that may or may not form a loop, with a loop shown in Fig. 1 and the loopless arrangement shown in Fig. 2. The strips are qualified as being flat as they have main surfaces having a width and a length substantially greater than a thickness. The "flat" nature of the elongated strips does not exclude the strips from being curved as they may be deformable, nor does it exclude them from containing holes or cutouts, or being constructed of a mesh, web or wireframe structure, so long as they functionally cover a sufficient area between the user's cheeks and the user's hands applying the cheek support.

Due to the location of the user support interface 90 relative to the casing 80 and to the user port 40, the hands of the self-user are positioned face to face with his or her respective cheeks, for the hands to be ergonomically applied against the cheeks and mouth floor when the self-user's mouth and/or nose is connected to the user port 40 (e.g., directly on the port 40 or on the mouthpiece). In this arrangement, the grips 90 (or portions thereof) are sandwiched between the hands and the cheeks and/or mouth floor, so that the weight of the device 10 is supported by the grips 90 or like user support interface. The grips 90 may be sufficiently flexible, for example in elastic deformation, in the dimensions normal to the skin surface in order to adapt to the unique shape of each user's face. The grips 90 may also be made of a material with shape retention capacity, i.e., the grips 90 can retain the shape they are deformed to, with a range of deformation. The grips 90 may be sufficiently rigid in a vertical dimension to support the weight of the device 10 and offer a stable position to the casing 80 relative to the user's mouth when supported in the manner shown in Fig. 2.

In accordance with an embodiment, the device 10 may be adequately supported by a single one of the grips 90. In accordance with another embodiment, the user support interface(s) 90 project in a same direction as the user port 40, and are vertically positioned on the casing 80 as a function of the position of the user port 40. For example, when the device 10 is in the orientation of Fig. 2, i.e., with the grips 90 and/or an axis of the user port 40 being generally horizontal, the grip(s) 90 or like user support interface 90 have their top edge being located level with the axis of the user port 40 or up to 5.0 cm above or 5.0 cm below the axis of the user port 40. As other possibilities, grips 90 could be mounted essentially around the mouthpiece or user port 40, i.e. the mouthpiece extrudes from the grip 90 or is even the same part. Grips 90 or like user support interfaces 90 may also go under the chin rather than on the cheeks, with the hands still supporting the cheeks. The user support interfaces 90 could also be in the shape of gloves connected to the casing 80.

Accordingly, the user support interface 90, such as the grips of Fig. 2, do *not per se* apply pressure on the cheeks or substantially contribute to supporting the soft tissues of the upper airways at all, as such support is substantially provided by the user's hands, over and below the grips 90, and by backing the grips 90. As the grips 90 are not configured to substantially apply pressure on the self-user's face, they have a height that may be at most 4.0 cm (though some grips could be more than 4.0 cm, such as glove-like user support interfaces 90) in a face contacting end. However, the height of at most 4.0 cm is substantially larger than a thickness of the grips 90. In accordance with an embodiment, the height to thickness ratio of the grips 90 is of at least 10, though it may be lower. This ratio may ensure that the grips 90 or like user interface 90 preserves the verticality of the device 10 as in Fig. 2, while being deformable or flexible to conform to the cheeks of the self-user. The capacity to deform or the flexibility may be expressed in terms of the handles 90 remaining in the elastic deformation when used. The capacity to deform or the flexibility could also imply some plastic deformation, as the handles 90 may be floppy in different manners. The purpose of the grips is to support the oscillometry device 10, in other words, to enable the self-user to use his or her hands simultaneously for manually applying cheek support and to manipulate, position and support the weight of the device 10 while putting his or her mouth on the user port 40. As such, the user support interface 90 must be pliable and have little rigidity in a direction normal to the cheek skin surface.

In another way to describe the arrangement of the user support interface, the user port 40 has a central axis X, and a reference plane P at the end of the user port 40 closest to the user, the central axis X being normal to the reference plane P of the user port 40 (Fig. 3, but applicable to all embodiments). The handles 90 being positioned laterally of the central axis and vertically located at least partially in a range of +5 cm to -5.0 cm of the central axis. The handles 90 project beyond the reference plane P by at least 3.0 cm in the user direction. In an embodiment, the end of the handles are located in a range from 3.0 cm to 12.0 cm from the reference plane P. In another embodiment, a position adjustment mechanism 93 may permit a user-adjustable vertical positioning of the grips 90 relative to the user port 40 to facilitate optimal positioning for a given user's physiognomy. The position adjustment mechanism 93 may be in the form of a slot for movement of the grips 90, indexed connection points (e.g., holes, grooves, etc) for the grips 90, among other possibilities.

Referring to an embodiment of the device 10 shown in Fig. 3, the user support interface 90 is in the form of grips that may include one or more ergonomically shaped straps 91 that surrounds the hand, or one or more fingers, so that the device 10 is retained from vertical movement relation to the self-user's hands while bringing the user port 40 towards the mouth. In the illustrated embodiment, there are two straps 91, one for the four fingers and another for the index and middle finger. There may be a single strap 91 per grip 90, even one in total for both grips 90, if there are two grips 90. The strap 91 may be for any of the fingers, including a single finger. The strap 91 may for instance be an elastic and/or may be tightened to a desired pressure. The position of the strap 91 on the grip 90 may be adjustable, as described above with reference to 93. One of the grips 90 and straps 91 are shown in Fig. 3, but the right-hand side may have another grip 90 and straps 91 as mirror images of the left-hand side of Fig. 2. Moreover, as shown in Fig. 1, the oscillometry device 10 may have straps only. In a similar embodiment, the grips 90 may be shaped as mittens or gloves. Moreover, in embodiments having two grips, a cross-link or bridge between the grips to pass under the chin may exist, and such a cross-link may be adjustable.

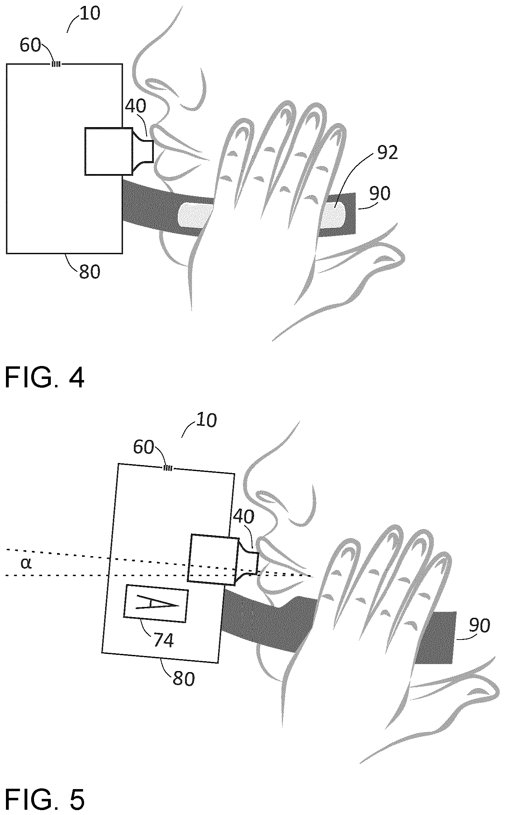

In the embodiment of the device shown in Fig. 4, the user support interface 90 may contain one or more sensors 92 to measure the pressure exerted by the user's hands on their cheeks and mouth floor, so that sufficient and adequately distributed cheek support can be assured. The output from the sensor(s) 92 may be used as a precondition or trigger to start a measurement, for instance by having the sensor(s) detect sufficient and adequately distributed cheek support. Moreover, the sensor(s) 92 could operate during oscillometry measurements to ensure that the cheek support is sufficient throughout the measurements. The sensors 92 may take any appropriate form, such as strain gauges.

In another embodiment, the user port 40 may include a concave space below the mouthpiece in which the user's tongue can comfortably be placed. Such a tongue space may be fitted with a sensor that can detect and confirm proper tongue placement, so that proper tongue placement may be used as a precondition or trigger to start a measurement. This feature may be in an oscillometry device or other lung function measuring device that is not necessarily self-use.

In another embodiment shown in Fig. 5, the oscillometry device 10 may include a sensor or sensor(s) 74 detecting the orientation of the oscillometry device 10 relative to a horizontal plane, so that correct orientation of the user facing slightly upwards may be asserted. The user may be guided to assume the correct position, e.g. by visual or audible signals, and proper device positioning may be used as a precondition or trigger to start a measurement. The sensor(s) 74 may for example be inertial sensors such as accelerometers.

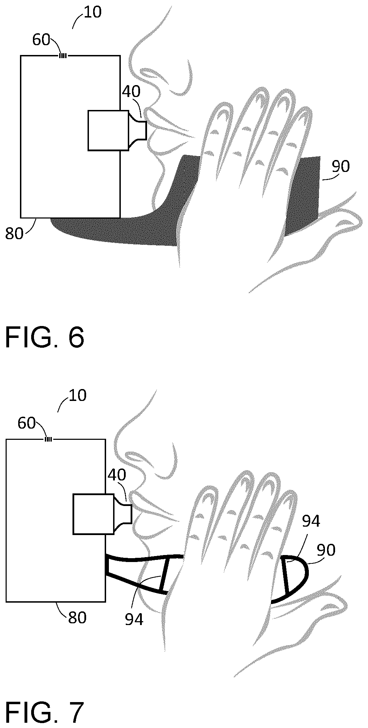

In another embodiment shown in Fig. 6, the user support interface 90 may be shaped as a single cup-shaped element attached to the lower portion casing 80 and designed to cradle the user's chin, again formed of sufficiently pliable material to adapt to the patient's facial shape in the presence of the user's hands providing cheek support. The cup-shaped element could be available in different sizes to be selected as a function of the user's anatomy.

In another embodiment shown in Fig. 7, the user support interface 90 may be implemented as a pliable wire frame element that spans the circumference of a flat, elongated grip area. In an embodiment, the pliable wire frame element may include additional support struts 94 to maintain the shape of the grip 90.

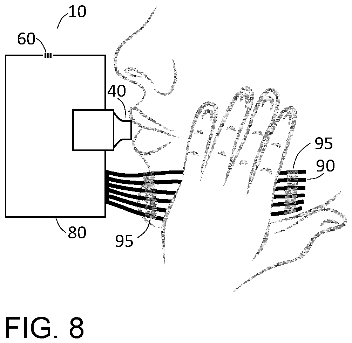

In another embodiment shown in Fig. 8, the user support interface 90 may be formed from a collection of individual pliable wires fanning out from the casing 80 in such a way that they collectively cover a flat, elongated area to between the user's hands and cheeks during cheek support. Again, one or more orthogonal connecting element 95 may be used to maintain the overall shape and integrity of this grip 90.

In accordance with an embodiment, the oscillometry device 10 has two or more of the items shown in Figs. 1-8 and described herein, to ensure a combination of proper cheek support, tongue placement and/or proper device orienting could be used as a precondition or trigger to start a measurement, possibly in combination with other criteria such as stable quiet breathing being detected by the device 10 before a measurement is automatically initiated. These features may be programmed into device 10, for example as part of the processing module 71.

In another embodiment, a cradle provided with the oscillometry device 10 may be shaped such that when the oscillometry device 10 rests on the cradle, the user support interface 90 is(are) naturally positioned such that the device 10 can easily be picked up by the user support interface(s) 90. The cradle may include means to charge the oscillometry device 10, which may include means for inductive charging, among other options.

In another example not covered by the appended claims of the device, the user support interface may be replaced by a head interface with elastic bands that are placed around the user's head so that the oscillometry device 10 can be worn like a mask, permitting entirely hands-free operation, e.g. to measure users on a treadmill or stationary bicycle during exercise testing. This feature may be in an oscillometry device that is not necessarily self-use.

## Claims

1. An oscillometry device (10) comprising:
a casing (80),
a user portion forming a conduit (20) entering into the casing (80), the conduit (20) adapted to receive a breath of a user,
an oscillometry measurement system operatively connected to the conduit (20) in the casing and adapted to produce oscillometry measurement signals from the breath of the user, and
a user support interface (90) projecting from the casing (80) in a common direction with the user portion, the user support interface (90) being made of a flexible deformable material, **characterized by** the user support interface including a pair of handles projecting from the casing and configured to cover cheeks of the user, the user support interface (90) configured to vertically support the oscillometry device (10) relative to a user when the user has his or her mouth on the user portion and his or her hands on the cheeks and the handles.

2. The oscillometry device (10) according to claim 1, wherein the handles are elongated strips of the flexible deformable material.

3. The oscillometry device (10) according to claim 2, wherein the elongated strips form loops.

4. The oscillometry device (10) according to claim 3, wherein the loops are sized to receive four fingers or a palm of a user.

5. The oscillometry device (10) according to claim 2, further comprising at least one finger loop on the lateral surfaces of the elongated strips.

6. The oscillometry device (10) according to claim 1, further comprising at least one pressure sensor on the handles configured to produce a signal indicative of a pressure applied thereon.

7. The oscillometry device (10) according to claim 1, further comprising at least one inertial sensor (74) on the casing (80) configured to produce a signal indicative of an orientation of the oscillometry device (10).

8. The oscillometry device (10) according to claim 1, wherein the handles have a height of at most 4.0 cm in a face contacting end.

9. The oscillometry device (10) according to claim 1, wherein a height to thickness ratio for the face contacting end of the handles is of at least 10.

## Patentansprüche

1. Oszillometrie-Vorrichtung (10), die Folgendes umfasst:
ein Gehäuse (80)
einen Anwenderteil, der eine Leitung (20) bildet, die in das Gehäuse (80) führt, wobei die Leitung (20) zur Aufnahme einer Atemluft eines Anwenders geeignet ist,
ein Oszillometrie-Messsystem, das funktionsfähig mit der Leitung (20) in dem Gehäuse verbunden ist und zur Erzeugung von Oszillometrie-Messsignalen aus der Atemluft des Anwenders geeignet ist, und
eine Anwenderhalterungsverbindung (90), die von dem Gehäuse (80) in einer gemeinsamen Ausrichtung mit dem Anwenderteil vorsteht, wobei die Anwenderhalterungsverbindung (90) aus einem flexiblen verformbaren Material hergestellt ist, **dadurch gekennzeichnet, dass**
die Anwenderhalterungsverbindung ein Paar Griffe einschließt, die von dem Gehäuse vorstehen und zur Bedeckung von Wangen des Anwenders konfiguriert sind, wobei die Anwenderhalterungsverbindung (90) zum vertikalen Halten der Oszillometrie-Vorrichtung (10) in Bezug auf einen Anwender konfiguriert ist, wenn der Anwender seinen oder die Anwenderin ihren Mund an den Anwenderteil und seine oder ihre Hände an die Wangen und die Griffe hält.

2. Oszillometrie-Vorrichtung (10) nach Anspruch 1, wobei die Griffe längliche Streifen aus dem flexiblen verformbaren Material sind.

3. Oszillometrie-Vorrichtung (10) nach Anspruch 2, wobei die länglichen Streifen Schlaufen bilden.

4. Oszillometrie-Vorrichtung (10) nach Anspruch 3, wobei die Schlaufen zur Aufnahme von vier Fingern oder einer Handfläche eines Anwenders dimensioniert sind.

5. Oszillometrie-Vorrichtung (10) nach Anspruch 2, die weiter mindestens eine Fingerschlaufe an den lateralen Oberflächen der länglichen Streifen umfasst.

6. Oszillometrie-Vorrichtung (10) nach Anspruch 1, die weiter mindestens einen Drucksensor an den Griffen umfasst, der zur Erzeugung eines Signals konfiguriert ist, das einen Druck, der darauf ausgeübt wird, anzeigt.

7. Oszillometrie-Vorrichtung (10) nach Anspruch 1, die weiter mindestens einen Trägheitssensor (74) an dem Gehäuse (80) umfasst, der zur Erzeugung eines Signals konfiguriert ist, das eine Ausrichtung der Oszillometrie-Vorrichtung (10) anzeigt.

8. Oszillometrie-Vorrichtung (10) nach Anspruch 1, wobei die Griffe eine Höhe von höchstens 4,0 cm in einem Gesichtskontaktende aufweisen.

9. Oszillometrie-Vorrichtung (10) nach Anspruch 1, wobei ein Verhältnis von Höhe zu Dicke für das Gesichtskontaktende der Griffe mindestens 10 beträgt.

## Revendications

1. Dispositif d'oscillométrie (10) comprenant :
un boîtier (80),
une partie d'utilisateur formant un conduit (20) pénétrant jusque dans le boîtier (80), le conduit (20) conçu pour recevoir le souffle d'un utilisateur,
un système de mesure d'oscillométrie relié de manière fonctionnelle au conduit (20) dans le boîtier et conçu pour produire des signaux de mesure d'oscillométrie à partir du souffle de l'utilisateur, et
une interface de support d'utilisateur (90) faisant saillie à partir du boîtier (80) dans une direction commune avec la partie d'utilisateur, l'interface de support d'utilisateur (90) étant fabriquée d'un matériau déformable flexible, **caractérisé en ce que** l'interface de support d'utilisateur inclut une paire de poignées faisant saillie à partir du boîtier et configurée pour couvrir les joues de l'utilisateur, l'interface de support d'utilisateur (90) configurée pour supporter verticalement le dispositif d' oscillométrie (10) par rapport à un utilisateur lorsque l'utilisateur a sa bouche sur la partie d'utilisateur et ses mains sur les joues et les poignées.

2. Dispositif d'oscillométrie (10) selon la revendication 1, dans lequel les poignées sont des bandes allongées du matériau déformable flexible.

3. Dispositif d'oscillométrie (10) selon la revendication 2, dans lequel les bandes allongées forment des boucles.

4. Dispositif d'oscillométrie (10) selon la revendication 3, dans lequel les boucles sont dimensionnées pour recevoir quatre doigts ou la paume d'un utilisateur.

5. Dispositif d'oscillométrie (10) selon la revendication 2, comprenant en outre au moins une boucle de doigt sur les surfaces latérales des bandes allongées.

6. Dispositif d'oscillométrie (10) selon la revendication 1, comprenant en outre au moins un capteur de pression sur les poignées configuré pour produire un signal indiquant une pression appliquée sur celles-ci.

7. Dispositif d'oscillométrie (10) selon la revendication 1, comprenant en outre au moins un capteur inertiel (74) sur le boîtier (80) configuré pour produire un signal indiquant une orientation du dispositif d'oscillométrie (10).

8. Dispositif d'oscillométrie (10) selon la revendication 1, dans lequel les poignées ont une hauteur d'au plus 4,0 cm dans une extrémité en contact avec le visage.

9. Dispositif d'oscillométrie (10) selon la revendication 1, dans lequel un rapport de la hauteur à l'épaisseur de l'extrémité en contact avec le visage des poignées est d'au moins 10.
